# EUROPÄISCHE PATENTANMELDUNG

(11) **EP 4 487 858 A1**
(43) Veröffentlichungstag der Anmeldung: **08.01.2025**
(21) Anmeldenummer: 23183553.9
(22) Anmeldetag: 05.07.2023
(51) Int. Cl.: A61K 35/644, A61K 31/12, A61K 31/201, A61K 31/23, A61K 36/63, A61K 36/899, A61K 36/9066, A61P 17/02

(54) **ZUSAMMENSETZUNG ZUR BEHANDLUNG VON WUNDEN**

(71) Anmelder: Karimi Tinat, Mansoureh, 44797 Bochum (DE)
(72) Erfinder: Karimi Tinat, Mansoureh, 44797 Bochum (DE)
(74) Vertreter: Schneiders & Behrendt Bochum

(57) **Zusammenfassung**

Die Erfindung betrifft eine Zusammensetzung zur Verwendung bei der Behandlung von Wunden, Geschwüren oder Verbrennungen, welche Honig, bei Raumtemperatur flüssige Fettsäuretriacylglyceride, wobei die Fettsäuren zumindest teilweise ungesättigte Fettsäuren sind, Curcumin und Getreidemehl enthält. Es hat sich herausgestellt, dass die erfindungsgemäße Zusammensetzung für die Behandlung von Wunden, insbesondere auch chronischen Wunden, besonders vorteilhaft ist und zu einer beschleunigten Heilung führt.

## Beschreibung

Die Erfindung betrifft eine Zusammensetzung zur Verwendung bei der Behandlung von Wunden, Geschwüren oder Verbrennungen, wobei die Zusammensetzung eine Honigbasis hat.

Die Verwendung von Honig zur Behandlung von Wunden ist bereits seit langer Zeit bekannt. Mit dem vermehrten Auftreten von Antibiotikaresistenzen hat die Verwendung von Honig zu Heilzwecken im Rahmen der Naturheilkunde in den letzten Jahren wieder zugenommen. Honig hat insbesondere eine entzündungshemmende Wirkung. Diese wird auf verschiedene Inhaltsstoffe des Honigs zurückgeführt, darunter Polyphenole und Flavone.

Durch seine antibiotische Wirkung hemmt Honig das Wachstum von Mikroorganismen in der Wunde, zudem regt es die Vermehrung von Immunzellen in der Wunde an.

Seit einigen Jahren werden Präparate zur Wundbehandlung auf Basis von Honig unter dem Namen Medihoney angeboten. Eine entsprechende Zusammensetzung auf Honigbasis wird in der DE 103 37 340 A1 beschrieben. Neben Honig enthält die Zusammensetzung insbesondere ethoxyliertes Süßmandelöl und Myristylmyristat. Das Süßmandelöl dient dabei als Tensid, während Myristylmyristat ein wachsartiges Material bildet. Durch die Hinzufügung von Süßmandelöl und Myristylmyristat soll eine in der Praxis besser handhabbare Zusammensetzung zur Verfügung gestellt werden. Insbesondere sorgt das Tensid für eine feine Verteilung der Wachsteilchen im Honig in Form einer Dispersion. Diese bereits auf dem Markt erhältlichen Präparate zur Wundbehandlung auf Honigbasis zeigen in der Regel zwar bereits eine gute Wirkung, die jedoch nicht in jedem Fall zufriedenstellend ist. Es stellte sich daher die Aufgabe, entsprechende Zusammensetzungen auf Honigbasis weiter zu verbessern.

Diese Aufgabe wird erfindungsgemäß gelöst durch eine Zusammensetzung zur Verwendung bei der Behandlung von Wunden, Geschwüren oder Verbrennungen, welche enthält:
- Honig,
- bei Raumtemperatur flüssige Fettsäuretriacylglyceride, wobei die Fettsäuren zumindest teilweise ungesättigte Fettsäuren sind,
- Curcumin und
- Getreidemehl.

Die Erfindung beruht auf der Erkenntnis, mehrere Komponenten miteinander zu kombinieren, wodurch eine für die Wundheilung besonders vorteilhafte Zusammensetzung geschaffen wird.

Beim eingesetzten Honig kann es sich um eine einzelne Honigsorte oder um ein Gemisch aus unterschiedlichen Honigen handeln. Besonders bevorzugt ist die Verwendung von Manuka-Honig, welcher Methylglyoxal enthält. Manuka-Honig kann aus dem Nektar der Südseemyrte gewonnen werden. Methylglyoxal wirkt antibaktieriell, beispielsweise gegen *Staphylococcus aureus, Escherichia coli* oder *Helicobacter pylori.* Manuka-Honig enthält deutlich mehr Methylglyoxal als andere Honigsorten; der Gehalt kann mehrere hundert Milligramm betragen.

Der Gehalt an Honig in der Zusammensetzung beträgt typischerweise 40 bis 70 Gew.-%, besonders bevorzugt 50 bis 70 Gew.-%. Besonders bevorzugt ist eine Zusammensetzung mit 40 bis 70 bzw. 50 bis 70 Gew.-% Manuka-Honig.

Unter Wunden im Sinne der Erfindung werden sämtliche Verletzungen von Haut oder Schleimhaut verstanden, unabhängig davon, ob sie Folge äußerer Einflüsse oder einer Krankheit sind. Im letzteren Fall spricht man auch von einem Geschwür, das auch als Ulcus bezeichnet wird. Wunden in Folge von äußeren Einflüssen können mechanisch, thermisch, chemisch oder durch Strahlung hervorgerufen werden. Bei mechanischen Wunden unterscheidet man u.a. Schnittwunden, Schürfwunden, Platzwunden oder Bisswunden. Bei thermischen Wunden spricht man auch von Verbrennungen, Verbrühungen oder Erfrierungen. Chemische Wunden können in Folge von Verätzungen auftreten, hervorgerufen insbesondere durch Säuren oder Laugen. Durch Strahlung hervorgerufene Wunden (aktinische Wunden) können auf radioaktive oder Röntgenstrahlung zurückgeführt werden. In der Praxis besonders häufig treten durch UV-Strahlung hervorgerufene Wunden auf, die man auch als Sonnenbrand bezeichnet.

Geschwüre (Ulcera) können u.a. auf mangelnde Durchblutung oder Infektionen zurückzuführen sein. Besonders Diabetiker und bettlägerige Menschen sind häufig von Geschwüren betroffen. Beispiele sind Ulcus cruris (offenes Bein), Dekubitus oder das diabetische Fußsyndrom. Erfindungsgemäß lassen sich insbesondere solche Geschwüre behandeln, die an Haut- und Schleimhäuten auftreten, welche von außen zugänglich und daher topisch behandelbar sind.

Die erfindungsgemäße Zusammensetzung kann insbesondere auch zusammen mit anderen Methoden der Wundbehandlung verwendet werden, beispielsweise im Zusammenhang mit dem Débridement. Hierunter versteht man die meist chirurgische oder mechanische Entfernung von abgestorbenem (nekrotischem) Gewebe aus der Wunde.

Die Zusammensetzung kann im Bereich der Human- oder Veterinärmedizin zum Einsatz kommen. In der Regel wird die Zusammensetzung topisch eingesetzt.

Der für die Herstellung der Zusammensetzung eingesetzte Honig oder auch die Zusammensetzung im Ganzen werden typischerweise sterilisiert, in der Regel mit Hilfe von Gammastrahlen. Auf diese Weise werden im Honig vorhandene Keime oder Schimmelpilze abgetötet, deren Eindringen in die Wunde verhindert werden muss.

Die Zusammensetzung enthält bei Raumtemperatur flüssige Fettsäuretriacylglyceride, die sich von Fettsäuren ableiten, bei denen es sich zumindest teilweise um ungesättigte Fettsäuren handelt. Soweit im Rahmen dieser Erfindung von Raumtemperatur die Rede ist, werden hierunter 25°C verstanden.

Als Fettsäuretriacylglyceride besonders bevorzugt sind Triacylglyceride der Ölsäure, bei der es sich um cis-9-Octadecensäure (C₁₈H₃₄O₂) handelt, d.h. eine einfach ungesättigte Fettsäure. Insbesondere kann es sich bei den Triacylglyceriden jedenfalls teilweise um Trioleine handeln, d.h. Triacylglyceride, bei denen es sich bei der Fettsäure in allen drei Positionen um Ölsäure handelt, also Glycerintrioleat.

Triolein ist in verschiedenen Ölen tierischen oder pflanzlichen Ursprungs enthalten, bspw. Olivenöl, Schmalz oder Mandelöl. Erfindungsgemäß besonders bevorzugt ist die Verwendung von Olivenöl. Dieses besteht aus Triacylglyceriden verschiedener Fettsäuren, wobei Ölsäure den Hauptanteil von ca. 75 % der Fettsäuren ausmacht. Weitere enthaltene Fettsäuren sind Palmitinsäure und Linolsäure.

Es hat sich herausgestellt, dass die in der Zusammensetzung enthaltenen flüssigen Fettsäuretriacylglyceride eine schmerzlindernde und kühlende Wirkung ausüben. Dies spielt insbesondere bei Brandwunden und schlecht heilenden Wunden eine Rolle.

Die positive Wirkung der Tatsache, dass es sich bei den Fettsäuren mindestens teilweise um ungesättigte Fettsäuren handelt, wird darauf zurückgeführt, dass diese eine antioxidative und entzündungshemmende Wirkung ausüben.

Der bevorzugte Gehalt an Fettsäuretriacylglyceriden, besonders bevorzugt an Olivenöl, beträgt 10 bis 30, bevorzugt 15 bis 25 Gew.-%.

Des Weiteren enthält die Zusammensetzung Curcumin ((1E,6E)-1,7-Bis(4-hydroxy-3-methoxyphenyl)-hepta-1,6-dien-3,5-dion). Curcumin ist eine intensiv gelbe Verbindung, die einen entscheidenden Bestandteil von Kurkuma darstellt. Kurkuma ist als Gewürz ein Bestandteil von Currypulver und darin für die gelbe Farbe verantwortlich. Auch die erfindungsgemäße Zusammensetzung kann Kurkuma selbst enthalten, dies ist sogar bevorzugt, weil Kurkuma neben Curcumin weitere relevante Inhaltsstoffe enthält. Darunter sind verschiedene Mineralien wie Kalium, Calcium, Magnesium, Eisen, Mangan, Selen und Zink. Ebenfalls enthalten sind Vitamin B₁ (Thiamin), Vitamin B₂ (Riboflavin), Vitamin B₃ (Niacin), Vitamin B₆ (insbesondere Pyridoxin), Vitamin C (Ascorbinsäure) und Vitamin E (Tocopherole). Die neben Curcumin enthaltenen Inhaltsstoffe entfalten eine positive Wirkung. Beispielsweise wirkt Zink antibakteriell und antientzündlich und steigert die Zellteilung in der Granulationsphase während der Wundheilung. In der Granulationsphase baut sich neues Bindegewebe auf, sodass sich die Wunde weiter schließt. Insbesondere hat sich herausgestellt, dass der Zusatz von Curcumin/Kurkuma für eine Heilung der Wunde in einer Weise sorgt, dass praktisch keine Narben zurückbleiben. Es gibt Hinweise, dass Curcumin/Kurkuma auch antibakteriell, antiviral und fungizid wirkt.

Eine wundheilende Wirkung von Curcumin bzw. Kurkuma ist grundsätzlich schon seit langer Zeit bekannt, nicht jedoch, dass sich in der erfindungsgemäßen Zusammensetzung zusammen mit Honig, Fettsäuretriacylglyceriden und Getreidemehl eine synergistische Wirkung ergibt.

Der Gehalt der erfindungsgemäßen Zusammensetzung an Kurkuma beträgt vorteilhafterweise 5 bis 10 Gew.-%. Alternativ kann bei Einsatz des wesentlichen Inhaltsstoffs Curcumin anstelle von Kurkuma Curcumin in einem Anteil von 0,1 bis 1, vorzugsweise 0,1 bis 0,5 Gew.-% eingesetzt werden.

Darüber hinaus hat sich herausgestellt, dass die Gegenwart von Getreidemehl die wundheilenden Eigenschaften der Zusammensetzung weiter verbessert. Besonders bevorzugt ist in diesem Zusammenhang Weizenmehl.

Getreidemehl hat feuchtigkeitsabsorbierende Eigenschaften. Ein Effekt der Verwendung von Getreidemehl ist daher, das überschüssiges Wundexsudat aufgenommen wird, wodurch die Wundheilung verbessert wird. Entsprechend kann das Getreidemehl den Heilungsprozess fördern und Schmerzen lindern. Zudem enthält Getreidemehl verschiedene für die Wundheilung bedeutsame Vitamine wie Vitamin B₁ (Thiamin), B₃ (Niacin) und B₅ (Pantothensäure). Vorzugsweise enthält die Zusammensetzung 10 bis 30, weiter bevorzugt 15 bis 25 Gew.-% Getreidemehl.

Die Zusammensetzung kann in unterschiedlicher Form vorliegen, typischerweise handelt es sich um eine Zusammensetzung, die sich gut auf eine Wunde auftragen lässt wie eine viskose Zusammensetzung. Insbesondere kann es sich um eine Salbe, ein Gel, eine Creme, ein Balsam oder eine Lotion handeln. Ebenso möglich ist die Verwendung als Bestandteil eines Wundverbands, eines Pflasters oder einer Bandage.

Die Zusammensetzung kann neben Honig, Fettsäuretriacylglyceriden, Curcumin und Getreidemehl weitere Bestandteile enthalten. Bevorzugt jedoch bilden diese vier Komponenten die vollständige Zusammensetzung, d.h. die Zusammensetzung enthält keine weiteren Komponenten. Es hat sich nämlich herausgestellt, dass die Autosterilität des Honigs für eine ausreichende Haltbarkeit der Zusammensetzung sorgt.

Beispiele für weitere Bestandteile der erfindungsgemäßen Zusammensetzung sind Tenside, die als Lösungsvermittler dienen, oder Konservierungsmittel, die die Haltbarkeit der Zusammensetzung verbessern. Typischerweise beträgt der Gehalt an Tensiden 2 bis 10 Gew.-%. Bevorzugt sind nichtionische Tenside.

Die erfindungsgemäße Zusammensetzung bewirkt eine beschleunigte Wundheilung und sorgt darüber hinaus für ein Verheilen von Wunden ohne bzw. mit reduzierter Narbenbildung. Durch die verbesserte Wundheilung wird auch die Eiterbildung reduziert, wodurch sich unangenehme, durch die Wunde hervorgerufene Gerüche reduzieren.

Neben der erfindungsgemäßen Zusammensetzung selbst betrifft die Erfindung auch die Verwendung der Zusammensetzung zur Behandlung von Wunden, Geschwüren und Verbrennungen, Verfahren zur Behandlung von Wunden, Geschwüren und Verbrennungen mit der Zusammensetzung sowie Verfahren zur Herstellung der erfindungsgemäßen Zusammensetzung. Sämtliche Ausführungen zur Zusammensetzung gelten in entsprechender Weise für die Verwendung, Anwendungs- und Herstellungsverfahren.

### Herstellungsbeispiel

Der Honig wird auf eine Temperatur von 40°C erwärmt, die weiteren Bestandteile der Zusammensetzung werden nach und nach in den Honig eingemischt. Die Zusammensetzung wird so lange auf 40 bis 50°C erhitzt, bis alle Bestandteile eine homogene Mischung bilden. Eine Erwärmung auf höhere Temperaturen sollte vermieden werden, um die Zersetzung wichtiger Bestandteile, insbesondere des Honigs, bspw. Enzymen, zu vermeiden. Die Zusammensetzung wird schließlich durch Gammastrahlung sterilisiert. Anschließend kann die Zusammensetzung als Salbe verwendet oder auf bzw. in einen Wundverband, ein Pflaster, eine Bandage o.ä. aufgebracht werden.

### Anwendungsbeispiele

Es wurden insgesamt 12 Patienten, die zumeist über mindestens zwei zu behandelnde Wunden verfügten, behandelt, einmal mit der erfindungsgemäßen Zusammensetzung und im Vergleich zumeist einmal mit einem herkömmlichen Hydrokolloidwundverband. Hydrokolloide Wundauflagen gehören zu den feuchten Wundauflagen, die ein gutes Wundheilungsmilieu mit Schutz vor Austrocknung gewährleisten sollen. Ein für die Heilung vorteilhaftes feuchtes Milieu wird bewahrt.
1) Eine 57jährige Frau, die beidseitig am diabetischen Fußsyndrom, Grad 3, litt, wurde mit einem Verband mit der erfindungsgemäßen Zusammensetzung und einem Hydrokolloidverband behandelt. Der mit der erfindungsgemäßen Zusammensetzung behandelte Fuß heilte in einer um ¼ verkürzten Zeit.
2) Eine 72jährige Frau mit mehreren Dekubituswunden, Grad 2-3, wurde mit einem Verband mit der erfindungsgemäßen Zusammensetzung und einem Hydrokolloidverband behandelt. Die mit der erfindungsgemäßen Zusammensetzung behandelte Wunde heilte in einer um 1/3 verkürzten Zeit.
3) Ein 80jähriger Mann mit Dekubitus, jeweils Grad 4, an der Ferse und an der Hüfte auf der linken Seite des Körpers, wobei die Wunden starke Exsudatbildung und eine Infektion zeigten, wurde mit einem Verband mit der erfindungsgemäßen Zusammensetzung (Ferse) bzw. mit einem Hydrokolloidverband (Hüfte) behandelt. Der Fuß heilte gegenüber der Hüfte in der Hälfte der Zeit.
4) Eine 35jährige Frau mit Verbrennungen am Unterarm, Grad 2, wurde mit der erfindungsgemäßen Zusammensetzung 3 Wochen lang behandelt. Die Wunden verheilten ohne auffällige Narbenbildung.
5) Eine 60jährige Frau mit Ulcus cruris, Grad 3, an beiden Beinen wurde an einem Bein mit einem Verband mit der erfindungsgemäßen Zusammensetzung, am anderen Bein mit einem Hydrokolloidverband behandelt. Der Zustand des mit der erfindungsgemäßen Zusammensetzung behandelten Beines verbesserte sich um ¼ schneller.
6) Ein 65jähriger Mann mit diabetischem Fußsyndrom an 2 Stellen am rechten Fuß wurde an einer Stelle mit einem die erfindungsgemäße Zusammensetzung aufweisenden Verband und an der anderen Stelle mit einem Hydrokolloidverband behandelt. Die mit der erfindungsgemäßen Zusammensetzung behandelte Stelle verbesserte sich in einer um 1/3 verkürzten Zeit im Vergleich zur anderen Stelle.
7) Ein 40jähriger Mann mit mechanischer Verbrennung, Grad 3, an der Schulter wurde mit der erfindungsgemäßen Zusammensetzung behandelt. Nach 1,5 Monaten waren die Verbrennungen geheilt mit lediglich geringer Narbenbildung.
8) Eine 70jährige Frau mit Ulcus cruris an beiden Beinen wurde am linken Bein mit einem die erfindungsgemäße Zusammensetzung tragenden Verband, am rechten Bein mit einem Hydrokolloidverband behandelt. Das linke Bein heilte zeitlich um ¼ schneller.
9) Eine 79jährige Frau mit Dekubitus, Grad 3, an Schulter und Hüfte auf der rechten Körperseite wurde mit einem die erfindungsgemäße Zusammensetzung tragenden Verband (Schulter) bzw. mit einem Hydrokolloidverband (Hüfte) behandelt. An der Schulter konnte ca. 1/3 schneller eine Verbesserung beobachtet werden als an der Hüfte.
10)Ein 9jähriges Mädchen mit einer Handverbrennung, Grad 2, wurde mit der erfindungsgemäßen Zusammensetzung behandelt. Nach 3 Wochen waren die Brandwunden geheilt mit lediglich unauffälliger Narbenbildung.
11)Ein 45jähriger Mann mit gebrochenem Bein nach Operation und nicht geheilter, infektiöser Wunde wurde mit der erfindungsgemäßen Zusammensetzung behandelt. Nach 2 Wochen war die Wunde mit lediglich kleinen Narben ausgeheilt.
12)Ein 76jähriger Mann mit beidseitigem diabetischen Fußsyndrom, Grad 3, wurde am rechten Fuß mit einem Verband, der die erfindungsgemäße Zusammensetzung trug, und am linken Fuß mit einem Hydrokolloidverband behandelt. Der rechte Fuß heilte um 1/3 schneller als der linke Fuß.

## Patentansprüche

1. Zusammensetzung zur Verwendung bei der Behandlung von Wunden, Geschwüren oder Verbrennungen, enthaltend
- Honig,
- bei Raumtemperatur flüssige Fettsäuretriacylglyceride, wobei die Fettsäuren zumindest teilweise ungesättigte Fettsäuren sind,
- Curcumin und
- Getreidemehl.

2. Zusammensetzung zur Verwendung nach Anspruch 1, **dadurch gekennzeichnet, dass** die Zusammensetzung Triacylglyceride der Ölsäure enthält.

3. Zusammensetzung zur Verwendung nach Anspruch 2, **dadurch gekennzeichnet, dass** die Zusammensetzung Triolein enthält.

4. Zusammensetzung zur Verwendung nach Anspruch 3, **dadurch gekennzeichnet, dass** die Zusammensetzung Olivenöl enthält.

5. Zusammensetzung zur Verwendung nach einem der Ansprüche 1 bis 4, **dadurch gekennzeichnet, dass** die Zusammensetzung 10 bis 30, bevorzugt 15 bis 25 Gew.-% der Fettsäuretriacylglyceride enthält.

6. Zusammensetzung zur Verwendung nach einem der Ansprüche 1 bis 5, **dadurch gekennzeichnet, dass** die Zusammensetzung 40 bis 70 Gew.-% Honig enthält.

7. Zusammensetzung zur Verwendung nach einem der Ansprüche 1 bis 6, **dadurch gekennzeichnet, dass** die Zusammensetzung Kurkuma enthält.

8. Zusammensetzung zur Verwendung nach Anspruch 7, **dadurch gekennzeichnet, dass** die Zusammensetzung 5 bis 10 Gew.-% Kurkuma enthält.

9. Zusammensetzung zur Verwendung nach einem der Ansprüche 1 bis 8, **dadurch gekennzeichnet, dass** das Getreidemehl Weizenmehl ist.

10. Zusammensetzung zur Verwendung nach einem der Ansprüche 1 bis 9, **dadurch gekennzeichnet, dass** die Zusammensetzung 10 bis 30, bevorzugt 15 bis 25 Gew.-% Getreidemehl enthält.

11. Zusammensetzung zur Verwendung nach einem der Ansprüche 1 bis 10, **dadurch gekennzeichnet, dass** der Honig Manuka-Honig ist.
